Europäisches Patentamt

European Patent Office

Office européen des brevets

(19)

(11) Publication number: 0 287 248
A2

# EUROPEAN PATENT APPLICATION

(21) Application number: 88302944.9

(51) Int. Cl.⁴ C12N 5/00

(22) Date of filing: 31.03.88

(30) Priority: 31.03.87 JP 79974/87

(43) Date of publication of application:
19.10.88 Bulletin 88/42

(84) Designated Contracting States:
CH DE FR GB LI

(71) Applicant: UBE INDUSTRIES, LTD.
12-32, Nishihonmachi 1-chome
Ube-shi, Yamaguchi-ken 755(JP)

(72) Inventor: Mori, Kazuhiro John Igarashi-chiku
Godoshukusha 1-102 7492-62 Ugarasgu
Bubinacgum Buugata-shi Niigata-ken(JP)
Inventor: Doi, Shoichi
31-6, Iwakura Nakaosagi-cho Sakyo-ku
Kyoto-shi Kyoto-fu(JP)
Inventor: Nishikori, Masaru
21, Ichijyoji Satonomae-choe Sakyo-ku
Kyoto-shi kyogo-fu(JP)
Inventor: Tezuka, Hiroaki
Rakusaitakenosato-danchi
14-202, 2-1 Oharanohigashitakenosato-cho
Nishikyo-ku Kyoto-shi Kyogo-fu(JP)

(74) Representative: Myerscough, Philip Boyd et al
J.A.Kemp & Co. 14, South Square Gray's Inn
London, WC1R 5EU(GB)

(54) A human cell line capable of producing human interleukin 3 having mast cell growth activity.

(57) A human cell line, or a clone or subclone thereof, each being capable of producing human interleukin 3 having mast cell growth activity, and a process for producing the human interleukin 3 having mast cell growth activity by culturing the human cell line, or the clone or subclone thereof, and recovering the resulting human interleukin 3 having mast cell growth activity.

EP 0 287 248 A2

# A HUMAN CELL LINE CAPABLE OF PRODUCING HUMAN INTERLEUKIN 3 HAVING MAST CELL GROWTH ACTIVITY

This invention relates to a human cell line, or a clone or subclone thereof, which produces human interleukin 3 having mast cell growth activity, and a process for producing human interleukin 3 having mast cell growth activity.

It is known that hematopoietic stem cells in bone marrow are differentiated into lymphocytes, granulocytes, monocytes, platelets and red blood cells essential to the maintenance and activity of life in living organisms, and play an important physiological role. It is known also that for differentiation of the stem cells, their proliferation is first of all important, and various factors are involved in their proliferation. Colony stimulating factor, and interleukin 3 (to be referred to as "IL-3") are among such factors. In particular, IL-3 is an important factor which acts on various blood progenitor cells in addition to pluripotent hematopoietic stem cells, and stimulates and induces proliferation and differentiation of various blood cells.

It is known that mouse IL-3 is produced in the conditioned medium of mouse spleen cells by lectin stimulation, or in the culture supernatant of a tumor cell line such as WEHI-3 [J. Immunol., 129, 1377 (1982)]. cDNA of mouse IL-3 was successfully cloned [Nature, 307 (19), 233 (1984)]. It was reported that human IL-3 is produced by transforming normal human T cells with human adult T cell leukemia virus (HTLV) [Science, 223, 703 (1984)]. Recently, it was reported that cDNA of human IL-3 was successfully cloned [Cell, 47, 3 (1986)].

The known factor which acts on undifferentiated T cells and induces formation of 20α-hydroxysteroid dehydrogenase (20α-SDH) and differentiates the cells into mature T cells was found to be the same as the mast cell growth factor (MCGF), and thus named "IL-3". Thereafter, cell lines FDCP-1 and -2 and DA-1 were established as cells which grow depending upon IL-3, and these cell lines have been used for assay of IL-3 activity.

Subsequently, it was found that formation of 20α-SDH is also induced by granulocyte macrophage colony stimulating factor (GM-CSF); and, FDCP-1 and -2 and DA-1 established as IL-3-dependent cells are also proliferated by interleukin 2 (to be referred to as IL-2) or GM-CSF. It was concluded therefore that 20α-SDH is not a specific marker for IL-3 and these cell lines cannot detect IL-3 specifically [Cellular and Molecular Biology and Immunology Today, 7, (4), 104 (1986)]. Thus, only MCGF activity seems to be a useful index for identifying IL-3 activity. Thus, excepting MCGF, substances previously considered as specific markers for IL-3 and used for measuring IL-3 activity were denied.

The methyl cellulose method previously known for the measurement of MCGF activity, however, cannot provide accurate information on MCGF activity because it permits measurement of macrophage colony stimulating factor (M-CSF) as well. Hence, no method which permits accurate measurement of MCGF activity has been known to date.

On the other hand, since IL-3 is a factor which acts on pluripotent hematopoietic stem cells, and stimulates and induces proliferation and differentiation of various blood cells, it is considered to have various CSF activities, i.e. multi-CSF activity. These properties of IL-3 have already been demonstrated with mouse IL-3, but have not yet been done with human IL-3 to date. For example, cloning of human IL-3 gene was successful [Cell, 47, 3 (1986)], but multi-CSF activity has not yet been demonstrated in this DNA clone.

It is an object of this invention to provide a human cell line capable of producing human interleukin 3 having mast cell growth activity.

According to this invention there is provided a human cell line capable of producing human interleukin 3 having mast cell growth activity.

According to this invention, interleukin 3 can be produced by a process which comprises culturing a human cell line, or a clone or subclone thereof, each being capable of producing human interleukin 3 having mast cell growth activity, and recovering the resulting human interleukin 3 having mast cell growth activity.

We have extensively worked on human cells which produce human interleukin 3 (to be referred to as human IL-3), and have succeeded in establishing a human leukemia T cell line (referred to as HIL-3) capable of producing human IL-3 having mast cell growth activity (to be sometimes referred to as MCGF activity) by culturing peripheral white blood cells of a patient with adult T cell leukemina (ATL) who showed a bone marrow pattern suggestive of chronic myelogenous leukemia (CML) and suffered from granulocytosis.

The HIL-3 established by the present inventors has been deposited under the terms of the Budapest Treaty with the Fermentation Research Institute, Agency of Industrial Science and Technology, Japan. It has the accession number FERM BP-1760 and can be cultured for a long period of time in media generally used to culture animal cells and optionally containing serum. The resulting human IL-3 can be obtained by known isolating and purifying methods such as salting out, dialysis and ultrafiltration. It can be purified to a higher purity by using ion-exchange chromatography, affinity chromatography, electrophoresis, etc. in combination with the above methods.

The resulting human IL-3 having MCGF activity which is produced as above is a novel factor which has not been reported previously. This factor can be produced by culturing the human leukemia T cell line established by the present inventors, and also other human cells capable of producing the above factor.

Sawada et al established an IL-3 producing mouse T-cell line (STIL-3) from C3H/HeN mice bearing radiation induced L8313 leukemia. Using STIL-3 culture supernatant and the methyl cellulose method known for assaying MCGF activity, bone marrow cells were cultured. Many macrophage colonies containing (or mixed with) mast cell colonies were detected. Therefore, the MCGF activity could not be accurately measured by the method as shown in Table 1.

## Table 1

### Frequency of mast cell progenitors in fresh bone marrow as assayed in semi-solid methyl cellulose culture

| Days of culture | Mast cell colonies | Granulocyte colonies | G-M colonies | Macro-phage-colonies | Plating efficiency (per 10 cells) |
|---|---|---|---|---|---|
| 7 | 0 | 73+5 | 41+8 | 219+11 | 0 |
| 14 | 15+2 | 0 | 18+2 | 270+17 | 15 |
| 21 | 11+2 | 0 | 0 | 198+10 | 11 |

They have extensively worked on an assay system for MCGF activity and finally established the following agar culture method by which completely pure mast cell colonies were formed with a seeding efficiency of 100-200 per $10^5$ bone marrow cells which was much higher than those previously reported. The results are shown in Table 2.

## Table 2

### Frequency of mast cell progenitors in fresh bone marrow as assayed in semi-solid agar culture

| Days of culture | Mast cell colonies | Granulocyte colonies | G-M colonies | Macro-phage-colonies | Plating efficiency (per 10 cells) |
|---|---|---|---|---|---|
| 7 | 67+3 | 0 | 3+1 | 11+2 | 67 |
| 14 | 137+8 | 0 | 0 | 0 | 137 |

Exactly the same results were obtained with subclones STIL-3A8 and STIL-3C5 derived from STIL-3.

The present invention will be described below in more detail.

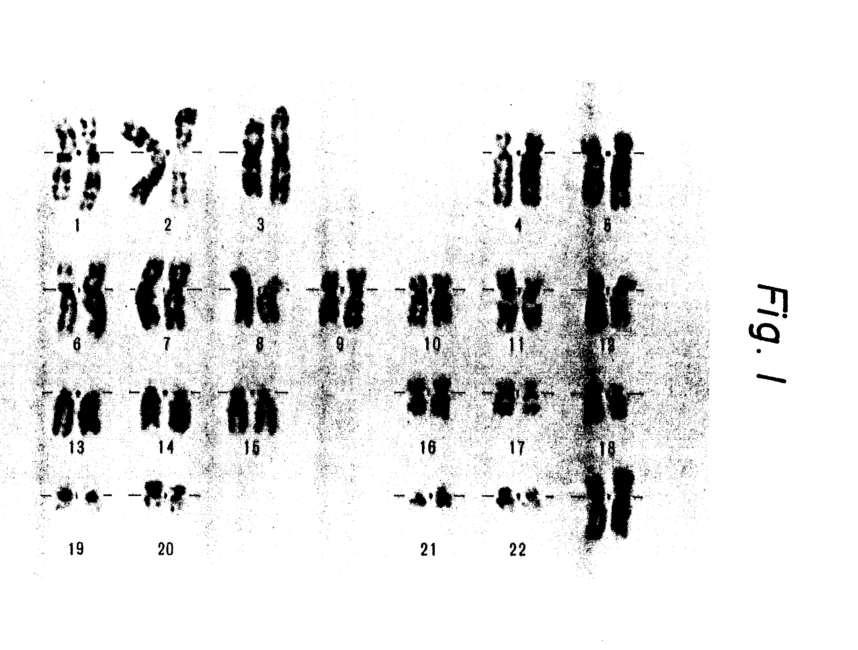

Figure 1 is a photograph showing an analysis of chromosomes of HIL-3.

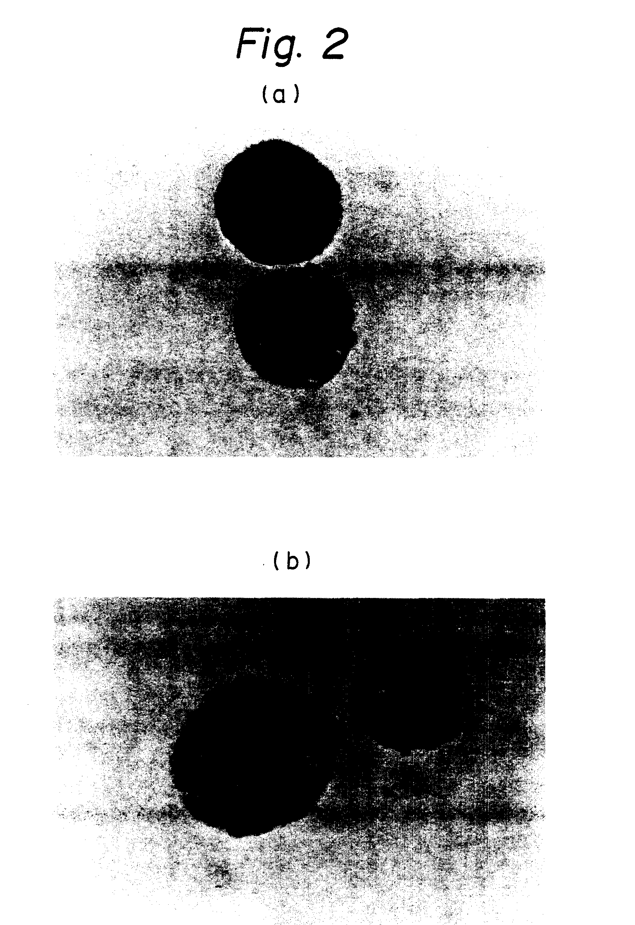

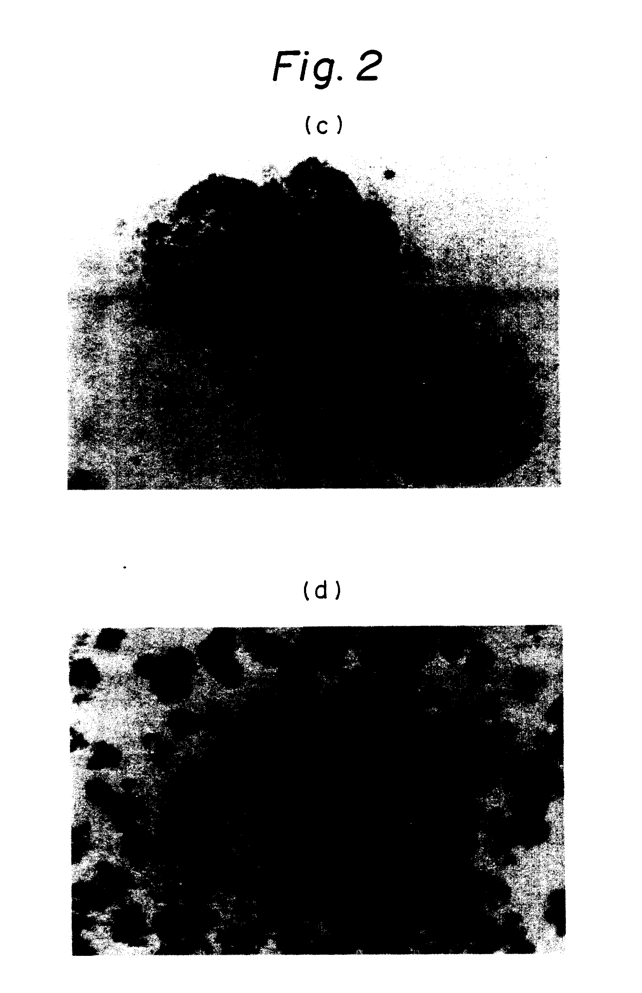

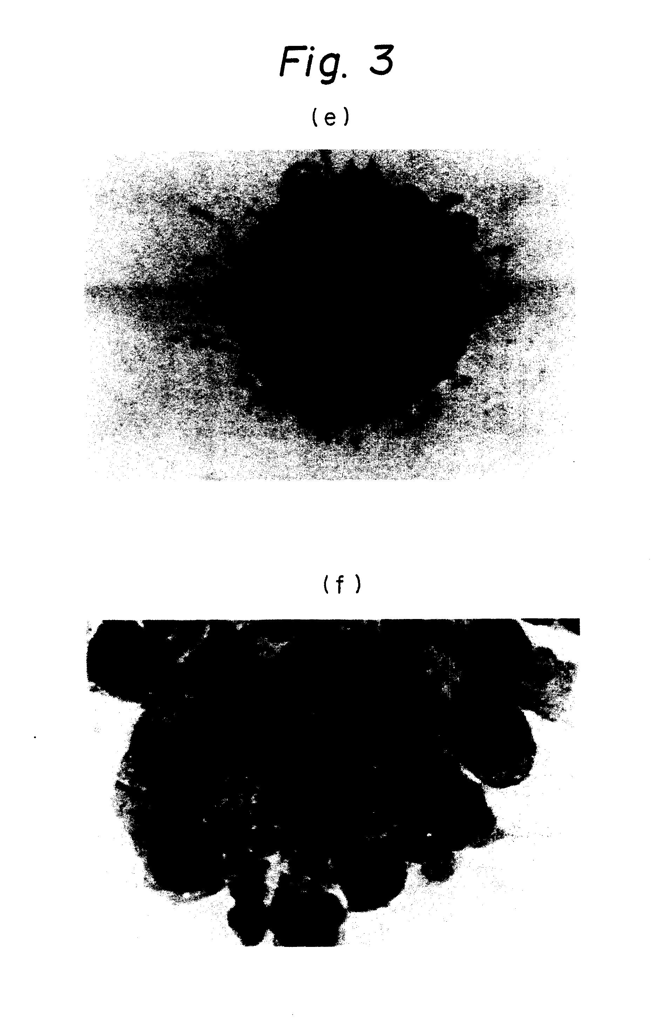

Figure 2 shows photographs of mouse mast cell colonies and human metachromatic cell colonies which are derived from mouse and human bone marrow cells respectively in the presence of HIL-3 (human T cell line) culture supernatant containing IL-3. In Figure 2, (a) is a photograph of mouse mast cells stained with toluidine blue; (b) is a photograph of mouse mast cells stained by the May-Grünwald-Giemsa method; (c) is a photograph of human metachromatic cells stained with toluidine blue; and (d) is a photograph of human metachromatic mast cell colonies stained with toluidine blue.

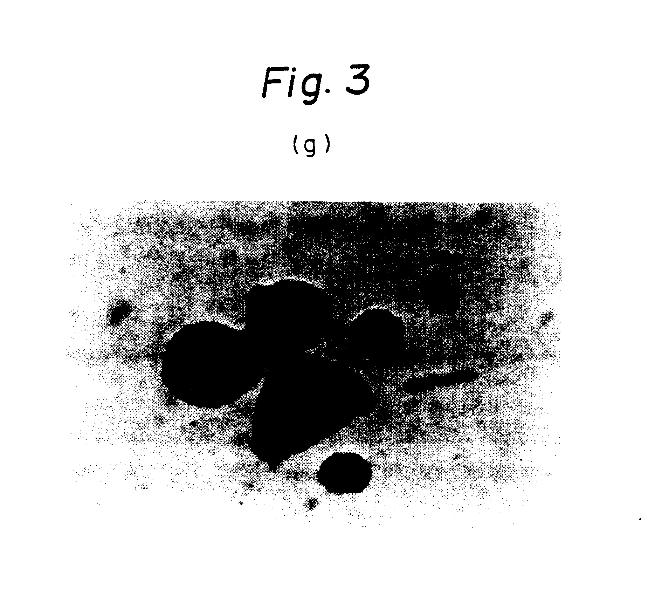

Figure 3 shows photographs of cells maintained in a suspension culture of human bone marrow cells in a medium containing the HIL-3 culture supernatant. In Figure 3, (e) is a photograph of megakaryocytes; (f) is a photograph of myeloblasts; and (g) is a photograph of basophilic cells, myeloblasts, proerythroblasts, etc.

## (1) Establishment of human IL-3-producing cell line

### (a) Isolation and establishment of HIL-3 cell line

The peripheral blood of a patient (a Japanese female 52 years old) with adult T cell leukemia accompanied by granulocytosis was taken, and monocytes were separated from it using Ficoll-Hypaque. The monocytes were cultured in RPMI-1640 medium containing 10% FCS and human IL-2 (2 U/ml) at 37 °C in a humidified atmosphere of 5% $CO_2$ in air. After about 1 month, proliferation of monocytes was observed, and on March 15, 1988 (the culture was started on September 9, 1986) the monocytes still showed continuous proliferation in the presence of 2U/ml of IL-2. Thus, a human T cell line (FERM BP-1760) capable of subcultivation was established.

### (b) Establishment of HIL-3 clone or subclone

Clones of the present cell line (HIL-3) could be obtained by selective cloning in a conventional manner. For example, by assaying the amount of IL-3 produced in each colony, several clones having high ability to produce IL-3 could be obtained.

Briefly stated, the original cells were plated to give 0.3 to 1 cell per well and cultured at 37 °C in air containing 5% $CO_2$, and then cell clones with high ability to produce IL-3 were selected.

## (2) Characteristics of human T cell HIL-3

### (a) Culture medium for maintaining and proliferating HIL-3

The present cell line could be well maintained and proliferated in RPMI-1640 medium containing 10% FCS and 1 to 2 U/ml of IL-2. It could also be maintained in an IL-2-free medium.

### (b) Maintenance and proliferation conditions for the cell line and its proliferation dependence

The present cell line well grows generally at 36 to 38 °C, preferably 37 °C, and a pH of 6.5 to 7.5, preferably 7.2. Preferably, it is cultured in an incubator containing 5% $CO_2$ and 95% air.

### (c) Cell proliferating ability

When the present cells ($2 \times 10^5$ cells/ml) are cultured under the aforesaid culture conditions, the cell density reaches at least $5 \times 10^5$ cells/ml after three days.

### (d) Subcultivation

The present cell line can be subcultured without limitation. At present after more than 1.5 years from the start, about the 90th subculture is being carried out.

(e) Storage in the frozen state

After being frozen to -80 °C under fixed conditions, the present cell line can be stored in a refrigerator (-80 to -100 °C) or in liquid nitrogen stably for a long period of time without a marked decrease in viable cells.

(f) Morphological characteristics

HIL-3 grows while forming aggregates composed of several tens of cells. The shape of the cells is medium to large with round and occasionally pseudopod-like protrusions, and is irregular.

The HIL-3 cell stained by the May-Grünwald-Giemsa method has a basophilic cytoplasm, and one or two round nuclei.

(g) Characteristics of chromosomes

The chromosomes are of the human female type. The structural abnormality common to the analyzed karyotypes was the inverted position of the long arm of the 14th chromosome, i.e. inv (14) (q11 q32). The karyotype was 46,XX,inv(14) (q11 q32).

(h) Determination by chromosome analysis

The HIL-3 cell line which showed most vigorous proliferation in the presence of IL-2 was treated with Colcemid for 60 minutes, treated with 0.075M KCl for 20 minutes, and fixed by the Carnoy solution. The chromosome specimen was spread over a slide glass by the air-drying method, photographed by a G-band and then analyzed.

Eight cells in the metaphase of division were analyzed with respect to karyotypes. All cells had inversion of the long arm of chromosome No. 14 [inv (14) (q11 q32)). This marker chromosome was malformed in six of eight cells. The remaining two cells further had two chromosomal malformations. Specifically, one had an extraneous chromosome X [ +X], and the other had 15q + chromosome [der(15) t (15;?) (Q26'?)]. The results are shown in Figure 1.

(i) Number of chromosomes      46, XX, inv (14) (q11 q32).

(j) Cell surface marker

All markers were observed under a fluorescent microscope by an indirect fluorescent antibody technique, and the rate of positive cells (%) was determined by visual observation.

It was found that the present cell line forms E-rossete, and was positive with respect to anti-human HLA-DR(HLA-DR), anti-T lymphocyte antibody (OKT1, OKT3), anti-helper T cell antibody (OKT4), anti-ED set receptor antibody (OKT11), and anti-IL-2 receptor antibody (Tac), and negative with respect to anti-suppressor T cell antibody (OKT8), anti-B cell antibody (B1) and anti-TdT antibody (TdT).

The results are shown in Table 3 below.

## Table 3

### Cell surface markers for HIL-3

| Phenotype | | |
|-----------|------|------|
| E-rossete | 84% | (+) |
| HLA-DR | 97% | (+) |
| OKT 1 | 95% | (+) |
| OKT 3 | 67% | (+) |
| OKT 4 | 79% | (+) |
| OKT 8 | 0% | (−) |
| OKT 11 | 96% | (+) |
| Tac | 95% | (+) |
| Bl | 0% | (−) |
| TdT | 0% | (−) |

These results led to the conclusion that the present cell line is a T cell line.


(3) IL-3 producing activity of the HIL-3 cell line

(a) Assay of IL-3 activity using mouse bone marrow cells as target cells

Bone marrow cells obtained from mouse femurs were converted to single cells in an α-medium by using a 26G needle. The bone marrow cells ($5 \times 10^4$ cells/ml) were added to the α-medium containing 35% horse serum (HS), 0.3% agar and 0 to 40% of HIL-2 culture supernatant. The culture mdium was then added to Petri dishes (made by Falcon Co.) with a diameter of 30 mm each in an amount of 2 ml, and cultured for 7 to 14 days at 37 °C in a humidified atmosphere of 5% $CO_2$ in air. The number of colonies was counted.

The results are shown in Table 4. The results are expressed as mean ±SD colony number/$10^5$ mouse bone marrow cells for three Petri dishes.

## Table 4

**Culture of mouse bone marrow in a semisolid agar medium in the presence of the HIL-3 culture supernatant**

| Concentration of HIL-3 culture supernatant (%) | 7 days | 14 days |
|---|---|---|
| 0 | 0 | 0 |
| 5 | 50±3.6 | 11±1.0 |
| 10 | 191±15.9 | 100±9.5 |
| 20 | 248±12.2 | 181±11.5 |
| 30 | 403±39.8 | 255±20.1 |
| 40 | 396±40.7 | 272±14.4 |

The results showed that the HIL-3 culture supernatant differentiated and induced about 80%, based on the total colonies, of mast cells on the 7th day, and 95%, based on the total colonies, of mast cells on the 14th day.

(b) Assay of IL-3 activity using human bone marrow cells as target cells

Normal human bone marrows were taken by an injection syringe through which heparin had been passed. Monocytes were separated from them by using Ficoll-Hypaque, and washed three times with $\alpha$-medium. The bone marrow cells adjusted to $5 \times 10^4$ cells/ml were inoculated in the $\alpha$-medium containing 25% HS, 0.3% agar and 5-30% HIL-3 culture supernatant, and cultured in Petri dishes (made by Falcon Co.). The culture was carried out in an incubator kept at a temperature of 37 °C in a humidified atmosphere of 5% $CO_2$ in air for 7 to 14 days, and then the number of colonies was counted.

The results are expressed as mean ± $SD/5 \times 10^4$ normal human bone marrow cells from four Petri dishes. The results are shown in Table 5.

## Table 5

### Culture of human bone marrow cells in a semisolid agar medium in the presence of the HIL-3 culture supernatant

| Concentration of HIL-3 culture supernatant (%) | 7 days | 14 days |
|---|---|---|
| 5 | 10±2.0 (*a) | NT |
| 10 | 16±1.2 | NT |
| 20 | 22±1.0 (*a) | 59±9.9 (*b) |
| 30 | 30±1.0 | NT |

(*a): 50% of the total colonies were meta-chromatic cell colonies stained with toluidine blue.

(*b): 90% of the total colonies were mast cell colonies.

NT: not tested

These results show that in human bone marrow cells, completely pure basophilic cells are produced at a rate of 100 to 400 colonies/$10^5$ human bone marrow cells.

The metachromatic cells obtained were perhaps mast cells.

Microphotographs of the mast cell colonies and metachromatic cell colonies produced by the HIL-3 culture supernatant are shown in (a), (b), (c) and (d) in Figure 2.

In Figure 3, (a) and (b) show specimens obtained by culturing the mouse bone marrow cells in a medium containing HIL-3 culture supernatant, preparing the cells of the resulting colonies into specimens using cytospin 2 (Shandon Inc.) and staining the specimens with toluidine blue and by the May-Gründwald-Giemsa staining method. (c) and (d) show specimens obtained by culturing human bone marrow cells in a medium containing HIL-3 culture supernatant, attaching the total culture medium including colonies to a slide glass on the 14th day, air-drying and fixing it, and staining it with toluidine blue.

Human bone marrow cells ($1 \times 10^5$ cells/ml) were added to the $\alpha$-medium containing 20% HS and 20% HIL-3 culture supernatant, and cultured. The culturing was continued at a temperature of 37 °C in a humidified atmosphere of 5% $CO_2$ in air, and every 7 days, half of the culture medium was replaced by a fresh one. It was consequently found that in the fourth week, as shown in Figure 3, cells such as megakaryocytes and myeloblasts could be maintained.

In Figure 3, (e) shows megakaryocytes in the fourth week of the culturing. (f) shows myeloblasts in the fourth week of the culturing. (g) shows proerythroblasts, basophilic cells and myeloblasts in the fourth week of culturing.

(d) Culturing of mouse bone marrow cells in a methyl cellulose medium in the presence of HIL-3 culture supernatant

Mouse bone marrrow cells ($1 \times 10^5$) were cultured in a culture medium containing 0.8% methyl cellulose, 30% FCS, 1% BSA, $1 \times 10^{-4}$M mercaptoethanol and HIL-3 culture supernatant not containing IL-2. As a result, hematopoietic progenitor cells (to be referred to as BFU-E) and pluripotent stem cells (to be referred to as CFU-Mix) were produced. The results are shown in Table 6. The results are expressed as mean ±SD colony/$1 \times 10^5$ bone marrow cells from three Petri dishes.

## Table 6

### Culture of mouse bone marrow cells in a methyl cellulose medium in the presence of HIL-3 culture supernatant

| Concentration of the supernatnat (%) | Colonies derived from hematopoietic progenitor cells | Colonies other than those from red blood cells |
|---|---|---|
| 0 | 6.7±1.2 | 20.7±8.1 |
| 10 | 9.7±2.1 | 201±20 |
| 20 | 6.7±0.6 | 220±30 |

The results show that HIL-3 culture supernatant not containing IL-2 also produces BFU-E and CFU-Mix.

The HIL-3 culture supernatant used in the present experiment was prepared by the following procedure.

HIL-3 cells growing in the presence of IL-2 were washed three times with PBS, and then cultivated for 3 days in a concentration of $1 \times 10^6$ cells/ml in RPMI-1640 medium containing 10% FCS and being free from IL-2, and the supernatant was taken. During this period, the proliferation of the cells stopped, but their surviving ability was maintained.

It was confirmed that the HIL-3 culture supernatant is produced with high activity and reproducibility for more than 3 days even after IL-2 was removed.

The human IL-3 having mast cell growth activity obtained by the present invention induces proliferation and differentiation of pluripotent stem cells and all other hematopoietic systems. The human IL-3 have proliferation stimulating activity not only on cells of the hemopoietic systems but also on other various cells. Accordingly, this human IL-3 is expected to be applicable to the treatment of hypoplastic anemia, thrombocytopenia, myelogenous lymphatic leukemia, erythremia, and side-effects of chemotherapy and radiation therapy. Particularly, it is used for the proliferation of bone marrow cells at the time of bone marrow transplantation.

Furthemore, by contrast to the conventional IL-3, the IL-3 in accordance with this invention induces proliferation of target cells and stem cells without decreasing the stem cells of bone marrow.

The present inventors succeeded in establishing a human T cell line having the ability to produce IL-3 which is expected to be applied to various conditions as mentioned above, and in producing human IL-3 from the T cell line.

## Claims

1. A human cell line, or a clone or subclone thereof, each being capable of producing human interleukin 3 having mast cell growth activity.

2. The human cell line, or its clone or subclone according to claim 1 wherein the human cell line is derived from a human leukemia T cell.

3. The human cell line or its clone or subclone according to claim 1 wherein the human cell line is human leukemia T cell line HIL-3.

4. A process for producing human interleukin 3 having mast cell growth activity, which comprises culturing a human cell line, or a clone or subclone thereof, each being capable of producing human interleukin 3 having mast cell growth activity, and recovering the resulting human interleukin 3 having mast cell growth activity.

5. The process of claim 4 wherein said human cell line, or clone or subclone thereof, each being capable of producing human interleukin 3 having mast cell growth activity is a human leukemia T cell line, or a clone or subclone thereof, each being capable of producing human interleukin 3 having mast cell growth activity.

# Fig. I

# Fig. 2

(a)

(b)

# Fig. 2

( c )

( d )

# Fig. 3

## ( e )

## ( f )

Fig. 3

(g)